**Europäisches Patentamt**

**European Patent Office**

**Office européen des brevets**

(19)

(11) Numéro de publication : **0 015 175**
**B1**

(12)

# FASCICULE DE BREVET EUROPÉEN

(45) Date de publication du fascicule du brevet :
09.06.82

(21) Numéro de dépôt : 80400123.8

(22) Date de dépôt : 25.01.80

(51) Int. Cl.³ : **A 61 K 35/72**

---

(54) **Médicament constitué de Pichia ou d'extraits de Pichia.**

---

Priorité : 25.01.79 FR 7901879

(43) Date de publication de la demande :
03.09.80 (Bulletin 80/18)

(45) Mention de la délivrance du brevet :
09.06.82 Bulletin 82/23

(84) Etats contractants désignés :
**CH DE GB IT**

(56) Document cité :
**FR - A - 2 374 042**

(73) Titulaire : **UNIVABLOT**
**21 bis rue Jonquoy**
**F-75014 Paris (FR)**

(72) Inventeur : **Massot-Claude, Jacqueline Olga**
**4 rue Prudhon**
**F-77340 Pontault-Combault (FR)**
Inventeur : **Astoin, Jacques Noel**
**5 rue Basse des Carmes**
**F-75005 Paris (FR)**

(74) Mandataire : **Casanova, André et al**
**Cabinet Casanova et Akerman 23 Boulevard de**
**Strasbourg**
**F-75010 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

# 0 015 175

## Médicament constitué de Pichia ou d'extraits de Pichia

La présente invention a pour objet l'utilisation de Pichia notamment Pichia fermentans, ou d'extraits de Pichia en tant que médicament.

Les Pichia sont des levures appartenant à la famille de Saccharomycetaceae et à la sous-famille des Saccharomycoideae qui comprend de nombreux genres. Ces genres sont définis par exemple dans « The Yeasts » (LODDER, North Holland Publishing Company, 1970, pages 405 à 554). L'espèce Pichia fermentans qui a été plus particulièrement étudiée par la Demanderesse, est connue et accessible dans plusieurs collections de micro-organismes, par exemple dans le CENTRAALBUREAU VOOR SCHIMMEL CULTURES (DELFT, Pays-Bas) sous le n° CBS 187.

A la connaissance de la Demanderesse, les Pichia n'ont jamais été étudiées en vue d'un usage thérapeutique. Des levures appartenant à un genre différent, tel que Saccharomyces cerevisiae, ont trouvé des applications thérapeutiques, en particulier comme protecteurs ou régénérateurs de la flore bactérienne intestinale.

Par ailleurs, certains auteurs ont étudié des extraits de Saccharomyces cerevisiae ou leurs glucanes dans leur action sur le système réticulo-endothélial (RIGGI et Di Luzio, Am. J. Physiol. (1961) *200*, 2, p. 297-300) ou dans leur action antitumorale (HAMURO et coll., C.A. (1978), *89*, 40 704 b).

Or, la Demanderesse a trouvé que les Pichia, notamment Pichia fermentans ainsi que ses extraits, en particulier les glucanes insolubles, possèdent des propriétés pharmacologiques exceptionnelles justifiant leur application en tant que médicaments des maladies infectieuses : immunostimulation non spécifique, potentialisation de l'action des antibiotiques et augmentation de l'activité des vaccins.

Les souches de Pichia peuvent être entretenues et cultivées de manière connue sur des milieux d'entretien et de fermentation habituellement utilisés pour les levures. Les cellules obtenues peuvent être séchées à 50 °C sous vide ou de préférence lyophilisées.

Les extraits selon l'invention, qui sont principalement des glucanes insolubles, peuvent être isolés de la paroi des cellules de Pichia, par exemple selon la méthode indiquée par Bell et Northcote (J. Chem. Soc. (1950), p. 1 944-1 947), éventuellement modifiée, par exemple selon Peat et coll. (idem, 1958a, p. 3 862-3 868). Le principe consiste à éliminer de la masse cellulaire obtenue par fermentation, d'abord les protéines et les lipides et les mannanes par traitement alcalin, puis le glycogène et les glucanes solubles par traitement acide.

Les exemples suivants illustrent l'obtention de ces produits.

### Exemple 1

### Procédé de fermentation de Pichia fermentans

a) Préparation des pieds de cuve.

On prépare le pied de cuve à partir de la culture d'entretien de la souche de Pichia fermentans. Celle-ci est obtenue par incubation pendant 48 heures à 30 °C de la levure sur pente gélosée contenant :

Milieu (1)

| | |
|---|---|
| eau de levure | 500 ml |
| peptone pancréatique | 2 g |
| glucose massé | 10 g |
| gélose | 18 g |
| eau ordinaire     qsq | 1 litre. |

On décolle la culture de la pente gélosée par environ 10 ml de sérum physiologique stérile par tube. On verse la suspension ainsi obtenue de manière stérile dans un erlenmeyer de 6 litres contenant 1,5 litre de milieu de fermentation ayant la composition :

Milieu (2)

| | |
|---|---|
| glucose | 10    g |
| extrait de levure | 2    g |
| sulfate d'ammonium | 5    g |
| phosphate monopotassique | 5    g |
| sulfate de magnésium (7$H_2$O) | 0,05 g |
| chlorure de calcium | 0,01 g |
| sulfate ferreux (7$H_2$O) | 0,01 g |
| chlorure de potassium | 0,01 g |
| eau ordinaire     qsq | 1 litre. |

L'incubation dure 24 heures à 27° ± 1 °C sur agitateur rotatif à environ 115 t/mn.

b) Fermentation.

On ensemence le contenu de 2 erlenmeyer tel qu'obtenu ci-dessus (environ 3 litres) dans une cuve de fermentation stérile remplie de 200 litres de milieu (2) décrit ci-dessus. On effectue la fermentation pendant 20 heures sous aération de 20 m³/heure. Ensuite on centrifuge le contenu de la cuve afin de récolter la masse contenant les cellules de Pichia.

On obtient 2,8 kg de masse cellulaire (humidité 74 %).

## Exemple 2

### Obtention de cellules desséchées de Pichia fermentans

En séchant à 50 °C sous vide 1 kg de la masse cellulaire obtenue dans l'exemple 1, on obtient 255 g de cellules prêtes à être utilisées.

Une variante, pour obtenir un produit plus facile à conserver et à utiliser, consiste à lyophiliser la masse cellulaire obtenue dans l'exemple 1 ; à partir de 1 kg de masse cellulaire on obtient ainsi 252 g de cellules utilisables.

## Exemple 3

### Obtention de glucanes de Pichia fermentans

On traite 1 kg de masse cellulaire fraîchement centrifugée obtenue selon l'exemple 1, par 3 litres d'une solution de soude à 6 % pendant 1 h 30 à 80 °C ; on recueille le résidu par centrifugation (4 000 t/mn pendant 15 mn) et on traite ensuite par 5 litres d'une solution de soude à 3 % pendant 18 heures à température ambiante ; on centrifuge à nouveau et on traite le produit insoluble de nouveau par une solution de soude à 3 % pendant 1 h 30 à 80 °C. On centrifuge et on reprend le culot de centrifugation par 3 litres d'eau. On amène la suspension ainsi obtenue à pH 4,5 par de l'acide acétique (ou chlorhydrique) et on porte à 80 °C pendant 2 heures sous agitation. On lave le résidu obtenu par centrifugation 3 fois par 1 litre d'eau bouillante et reprend par 2,5 litres d'une solution d'acétate de sodium 0,02 M puis procède à un autoclavage à 2 bars pendant 1 heure.

On lave la partie insoluble à l'eau, puis à l'éthanol, à l'acétone et on sèche sous vide à 50 °C maximum. On obtient après broyage du produit obtenu 15,5 g d'une poudre crème.

L'alternative consiste à lyophiliser ; à partir de la même quantité de cellules on obtient 14,2 g d'extrait.

Analyse : $C = 45,15\%$ ;  $H = 7,42\%$ ;  $O = 47,4\%$

$N < 0,2\%$ ;  $P$ : absence.

L'action de l'amylase d'origine pancréatique sur l'extrait ne libère que des traces de composés réducteurs, indiquant l'absence de glycogène.

L'extrait selon l'invention est remarquable par son insolubilité dans l'eau, les solutions alcalines ou acides, et les solvants organiques usuels, tels que l'éthanol, l'éther diéthylique, l'acétate d'éthyle, l'acétone, le chloroforme, etc.

On constate toutefois une très légère solubilité dans le diméthylsulfoxyde (DMSO).

Pouvoir rotatoire spécifique : $[\alpha]_D^{20} \simeq -60°$ (C = 0,2 ; DMSO).

Infrarouge : le spectre obtenu avec pastille de KBr présente une absorption caractéristique à 885 cm$^{-1}$.

Il ressort de ces données que les extraits obtenus de la paroi de Pichia fermentans sont principalement des polymères ramifiés de glucoses, liés par une majorité de liaisons β. L'action de l'exo-β-(1-3)-D-glucanase sur l'extrait libère des composés réducteurs confirmant ainsi l'existence de liaisons β-(1-3). L'extrait selon l'invention est donc constitué essentiellement de β-(1-3)-D-glucanes qui seront appelés simplement « glucanes » dans le texte.

Les levures Pichia, en particulier Pichia fermentans ainsi que les glucanes qui en sont extraits ont été soumis à des essais pharmacologiques.

### Toxicité

Aucune toxicité n'a été constatée chez la souris par voie orale, aussi bien pour Pichia fermentans que pour ses glucanes.

Par voie I.P., la DL$_{50}$ de ces glucanes est supérieure à 500 mg/kg.

### Mise en évidence de l'Immunostimulation par les cellules entières de Pichia et par ses glucanes

1. Cellules entières de Pichia fermentans per os.

Traitement préventif et curatif.

Les animaux utilisés sont des souris SPF IFFA CREDO, femelles, de 18-20 grammes, nourries avant l'expérience durant une semaine avec un régime ne comportant aucune levure mais contenant la dose de vitamines nécessaire. Les souris sont réparties en 2 lots de 10 animaux :
— 1 lot témoin d'infection ;
— 1 lot infecté et traité par Pichia.

Les animaux sont infectés par une souche très pathogène de Staphylococcus aureus (souche Institut Pasteur n° 54 146) par voie I.V., le Staphylococcus aureus utilisé est réactivé par passage sur souris avant l'essai. Une culture en bouillon nutritif incubée durant 16 heures à 37 °C est utilisée après dilution à 1/100 pour infecter les souris. Chaque souris reçoit par voie I.V. dans ces conditions environ $6 \times 10^5$ germes virulents.

Le schéma opératoire est le suivant :

|  | Lot témoin | Lot traité |
|---|---|---|
| Jour – 3<br>Jour – 2<br>Jour – 1 | 0,2 ml de sérum<br>physiologique<br>per os / souris | 0,2 ml de suspension<br>de Pichia à<br>3 g/20 ml per os/<br>souris |
| Jour – 0 | INFECTION STAPHYLOCOCCIQUE PAR VOIE I.V. | |
| Jour 0 à<br>Jour + 7 | idem J-3 à J-1 | idem J-3 à J-1 |

Le nombre de souris mortes dans chaque lot est noté chaque jour.

Cet essai a été effectué 5 fois et a donné les résultats suivants exprimant le nombre de souris survivantes.

|  | Lot témoin | Lot traité |
|---|---|---|
| essai n° 1 | 1/10 | 3/10 |
| essai n° 2 | 3/10 | 10/10 |
| essai n° 3 | 1/10 | 2/10 |
| essai n° 4 | 3/10 | 1/10 |
| essai n° 5 | 0/10 | 6/10 |
| % de survie | 16% | 44% |

Comme on peut le constater le nombre de souris mortes dans le lot traité est inférieur au nombre de souris mortes dans le lot témoin.

Etant donné la virulence du germe pathogène injecté, la protection conférée par l'administration per os de Pichia est importante.

Cette protection est attribuable à une immunostimulation non spécifique.

2. Cellules entières de Pichia et ses glucanes par voie I.P.

Traitement préventif.

Les animaux utilisés sont des souris SPF IFFA CREDO, femelles, de 18-20 grammes, nourries avant expérience durant 8 jours avec un régime dépourvu de levures mais contenant la dose de vitamines nécessaires.

Ces souris sont réparties en 2 lots :
— 1 lot témoin d'infection ;
— 1 lot infecté et traité soit par Pichia soit par ses glucanes.

Les expériences ont été effectuées sur les souris infectées par voie I.V. par environ $1 \times 10^6$ Staphylococcus aureus pathogènes (souche institut Pasteur, n° 54146) préparés comme décrit précédemment.

Le traitement a été effectué par voie I.P. :
— pour Pichia fermentans à la dose de 50 mg/kg,
— pour les glucanes de Pichia fermentans à 10 mg/kg.

4

**0 015 175**

Le schéma opératoire est le suivant :

|  | Lot témoin | Lot traité |
|---|---|---|
| Jour - 7 | Injection I.P. de O,2 ml de sérum physiolo-gique par souris | Injection I.P. de O,2 ml de suspension de Pichia ou de glu-canes |
| Jour - 4 | 2ème injection idem | 2ème injection idem |
| Jour  O | infection staphylococcique voie I.V. | |

Le nombre de souris mortes dans chaque lot est noté chaque jour.
Les tableaux suivants indiquent le nombre d'animaux survivants constaté sur plusieurs essais.

A. Avec levures entières de Pichia fermentans.

|  | Lot témoin | Lot traité |
|---|---|---|
| essai N° 1 (sur 10 souris) | 2 | 6 |
| essai N° 2 (sur 5 souris) | 1 | 5 |
| essai N° 3 (sur 10 souris) | O | 8 |
| % de survie | 12% | 76% |

B. Avec les glucanes de Pichia.

|  | Lot témoin | Lot traité |
|---|---|---|
| essai N° 1 (sur 10 souris) | 1 | 9 |
| essai N° 2 (sur 5 souris) | O | 3 |
| essai N° 3 (sur 10 souris) | O | 8 |
| essai N° 4 (sur 10 souris) | 2 | 7 |
| % de survie | 8,5% | 77,2% |

Comme on peut le constater, le nombre de souris mortes est inférieur dans les lots traités au nombre de souris mortes dans les lots témoins que ce soit avec les cellules entières de Pichia fermentans ou avec ses glucanes.

Etant donné la virulence du germe injecté, la protection conférée par l'injection I.P. de Pichia ou de

5

## 0 015 175

ses glucanes est très importante.

Cette protection est attribuable à une immunostimulation non spécifique.

Mise en évidence de l'immunostimulation par les glucanes de Pichia avec injection préalable d'Antigène

Les animaux utilisés sont des souris SPF IFFA CREDO, femelles, de 18-20 grammes, nourries avant l'expérimentation durant une semaine avec un régime ne comportant aucune levure mais contenant la dose nécessaire de vitamines.

Ces souris sont réparties en 4 lots de 10 :
— lot 1 : témoin d'infection,
— lot 2 : témoin antigène (« vaccin »),
— lot 3 : recevant l'antigène spécifique + infection,
— lot 4 : idem, lot 3, avec en supplément un traitement de glucanes de Pichia.

L'antigène est ici constitué d'une culture de Staphylococcus aureus (Institut Pasteur 54 146) à dose non mortelle.

La culture de 16 heures en bouillon nutritif est diluée au moment de la vaccination à $1 \times 10^{-4}$, représentant environ $1 \times 10^4$ germes/ml.

Les souris des lots 1, 3 et 4 sont infectées par la souche très pathogène de Staphylococcus aureus (Institut Pasteur 54 146) réactivée. Une culture en bouillon nutritif, incubée durant 16 heures à 37 °C est injectée sans dilution par la voie I.V. pour infecter les souris (soit $1 \times 10^8$ germes/ml).

Le schéma opératoire est le suivant :

| | Lot 2 témoin ANTIGENE | Lot 1 témoin d'infection | Lot 3 vacciné + infecté | Lot 4 vacciné + infecté + GLUCANES |
|---|---|---|---|---|
| Jour - 9 | 0,2 ml I.V. de $10^4$ germes/ml | 0,2 ml I.V. de sérum physiologique | 0,2 ml I.V. de $10^4$ germes/ml | 0,2 ml I.V de $10^4$ germes/ml |
| Jour - 6<br>Jour - 5<br>Jour - 4<br>Jour - 3<br>Jour - 2<br>Jour - 1 | 0,2 ml I.V. de sérum physiologique par jour | 0,2 ml I.V. de sérum physiologique par jour | 0,2 ml I.V. de sérum physiologique par jour | 0,2 ml I.P. de 10 mg/kg/ jour de Glucanes |
| Jour - 0 | 0,2 ml I.V. de sérum physiologique | infection par voie I.V.<br><br>(0,2 ml de culture à $10^8$ germes/ml) | | |

Le nombre de souris mortes dans chaque lot est noté chaque jour. Le tableau suivant indique les survies obtenues au cours du temps.

| JOURS APRES INFECTION | Lot 1 témoin infection | Lot 2 témoin Antigène | Lot 3 Antigène + infection | Lot 4 Antigène + Infection + Glucanes |
|---|---|---|---|---|
| 1er jour | 0/10 | 10/10 | 9 | 10 |
| 2ème jour | | | 7 | 9 |
| 3ème jour | | | - | - |
| 4ème jour | | | 3 | 3 |
| 5ème jour | | | - | - |
| 6ème jour | | | 1 | - |
| 7ème jour | | | - | - |
| 8ème jour | | | 0 | - |
| Fin d'essai | 0/10 | 10/10 | 0/10 | 3/10 |

6

100 % des animaux infectés par une dose mortelle de Staphylococcus aureus sont morts en 24 heures.

Les animaux, s'ils ont été vaccinés une fois 9 jours avant l'infection mortelle sont légèrement protégés puisqu'ils ne meurent à 100 % qu'au bout de 8 jours.

Comme on peut le constater ici, l'addition à la vaccination d'un traitement par les glucanes par la voie I.P. protège les animaux non seulement par l'allongement du temps de survie mais encore par l'abaissement du taux des mortalités.

Etant donné l'extrême virulence du germe injecté, la potentialisation conférée par les glucanes à une pré-vaccination spécifique a été importante. Cette potentialisation est là encore attribuable à une immunostimulation.

Mise en évidence de la potentialisation de l'action des antibiotiques

Dans cet essai, on a adjoint à un traitement antibiotique à court terme [pénicilline 1 seule injection], mais à dose thérapeutique normale (30 000 unités/kg), un traitement curatif de 2 jours par des glucanes à la dose de 10 mg/kg.

Les animaux utilisés sont de la même souche qu'aux essais précédents et ont le même régime alimentaire.

Les souris sont réparties en 4 lots de 5 souris :
— lot 1 : témoin d'infection,
— lot 2 : traité par glucanes de Pichia,
— lot 3 : recevant 1 injection de pénicilline,
— lot 4 : recevant — glucanes de Pichia — 1 injection de pénicilline.

L'essai a été effectué sur des souris infectées par voie I.V. par environ $1 \times 10^6$ Staphylococcus aureus pathogènes (I.P. 54 146) réactivés.

Une culture en bouillon nutritif incubée durant 16 heures à 37 °C est utilisée après dilution au 1/100 pour infecter les souris.

Le schéma opératoire est le suivant :

|  | Témoin d'infection | Glucanes | Pénicil-line | Penicilline Glucanes |
|---|---|---|---|---|
| Jour O | INFECTION | STAPHYLOCOCCIQUE | VOIE | I.V. |
| Jour + 1 | rien | I.P. : 0,2 ml suspension glucanes 10 mg/kg | I.M. : 0,2 ml solution pénicil-line 30.000 UI/ kg | I.P. : 0,2 ml sus-pension glucanes 10 mg/kg + I.M. 0,2 ml solution pénicilli-ne 30.000 UI/ kg |
| Jour + 2 | rien | idem | rien | IP : glucanes |

Le nombre de souris mortes est relevé chaque jour. Le tableau suivant indique les survies observées pour chaque lot.

| Témoins | Glucanes seuls | Pénicilline seule | Glucanes + Pénicilline |
|---|---|---|---|
| 1/5 | 3/5 | 2/5 | 4/5 |

Comme précédemment, il y a eu protection par les glucanes de Pichia seuls bien qu'utilisés dans cet essai en traitement curatif (3/5).

La pénicilline a faiblement protégé les animaux (2/5).

L'adjonction de glucanes à cette même dose de pénicilline a potentialisé l'action antibiotique de la pénicilline (4/5).

Les essais relatés ci-dessus ont mis en évidence que l'administration de Pichia fermentans ou de ses glucanes d'une part diminue par immunostimulation non spécifique la mortalité d'animaux infectés par un germe pathogène et, d'autre part, potentialise l'action d'antibiotiques et celle de vaccins. Cette levure est donc susceptible d'être utilisée pour renforcer la résistance de l'organisme contre les agents infectieux et pour améliorer les défenses immunitaires.

Les levures Pichia fermentans et les glucanes qui en sont extraits étant dépourvus de toxicité peuvent donc être utilisés dans la prévention et/ou le traitement des maladies infectieuses aiguës ou chroniques, en particulier des maladies de la sphère ORL et/ou pulmonaire, mais aussi de toute autre maladie d'origine virale ou bactérienne.

La Pichia fermentans et ses glucanes peuvent être utilisés comme médicaments seuls ou en association avec un ou plusieurs antigènes ou encore en association avec un ou plusieurs antibiotiques.

Les cellules entières de pichia peuvent être administrées par voie orale à des doses quotidiennes de 100 à 2 000 mg (produit sec) par jour, de préférence dans des gélules contenant les cellules lyophilisées et éventuellement des excipients usuels, ou bien en ampoules buvables, les cellules étant en suspension dans leur milieu de culture.

Exemple de composition d'une gélule

| | |
|---|---|
| — Cellules lyophilisées de pichia fermentans | 50 mg |
| — Lactose | 7 mg |
| — Stéarate de magnésium | 2 mg |
| — Sucre glace    qsp une gélule de | 150 mg |

Les glucanes de Pichia peuvent aussi être administrés par voie orale, à des doses quotidiennes de 20 à 500 mg, ou seront utilisés également sous forme de suspensions injectables, à raison de 5 à 100 mg par jour. On peut aussi envisager l'usage de ces glucanes sous forme de crèmes dermiques ou d'aérosols.

Exemple de composition de suspension injectable

| | | |
|---|---|---|
| — Glucanes de Pichia fermentans (*) micronisés | 0,010 | g |
| — Polysorbate 80 | 0,025 | g |
| — Polyvinylpyrrolidone | 0,025 | g |
| — Phosphate monosodique | 0,025 | g |
| — Chlorure de sodium qsp isotonie | | |
| — Merthiolate de sodium | 0,000 2 | g |
| — Eau distillée apyrogène qsp | | |
| — 1 ampoule injectable de 5 ml | | |

(*) (particules < 50 µm dont 80 % < 10 µm).

Exemple d'aérosol

| | | |
|---|---|---|
| — Glucanes micronisés (*) | 0,050 | g |
| — Polysorbate 80 | 0,2 | g |
| — Glycérides oléiques polyoxyéthylènes | 1 | g |
| — Phosphate monosodique | 0,1 | g |
| — Chlorure de sodium qsp isotonie | | |
| — Merthiolate de sodium | 0,000 8 | g |
| — Eau purifiée    qsp | 20 | ml. |

**Revendications**

1. Substance constituée de Pichia ou d'extraits de Pichia pour le traitement des maladies infectieuses, immunostimulation non spécifique, potentialisation de l'action des antibiotiques et augmentation de l'activité des vaccins.

2. Substance selon la revendication 1, caractérisée par le fait qu'elle est constituée de Pichia fermentans ou d'extraits de Pichia fermentans.

3. Substance selon la revendication 2, caractérisée par le fait qu'elle est constituée de cellules entières de Pichia fermentans.

4. Substance selon la revendication 2, caractérisée par le fait qu'elle est constituée d'extraits de paroi de Pichia fermentans obtenus par un procédé consistant à éliminer des cellules de Pichia fermentans, les protéines, les lipides et les mannanes par traitement alcalin, puis le glycogène et les glucanes solubles par traitement acide.

5. Substance à base d'extraits selon les revendications 2 ou 4, caractérisée par le fait que les extraits sont principalement constitués de glucanes insolubles.

6. Substance selon la revendication 5, caractérisée en ce que les glucanes insolubles sont essentiellement des β-(1-3)-D-glucanes.

7. Composition pharmaceutique renfermant à titre de substance active de la Pichia ou des extraits de Pichia tels que définis dans l'une quelconque des revendications 1 à 6, ainsi qu'un véhicule pharmaceutiquement acceptable.

8. Composition pharmaceutique selon la revendication 7, administrable par voie orale, caractérisée par le fait qu'elle contient des cellules entières de Pichia ou d'extraits de Pichia.

9. Composition pharmaceutique selon la revendication 7, administrable par voie parentérale, caractérisée par le fait qu'elle contient des glucanes insolubles de Pichia.

10. Composition pharmaceutique selon la revendication 7, sous forme d'aérosol, caractérisée par le fait qu'elle contient des glucanes insolubles de Pichia.

## Claims

1. Material composed of Pichia or of extracts of Pichia for the treatment of infectious diseases, nonspecific immunostimulation, potentiation of the action of antibiotics and increasing of the activity of vaccines.

2. Material according to claim 1, characterised by the fact that it is composed of Pichia fermentans or of extracts of Pichia fermentans.

3. Material according to claim 2, characterised by the fact that it is composed of whole cells of Pichia fermentans.

4. Material according to claim 2, characterised by the fact that it is composed of extracts of wall of Pichia fermentans obtained by a process consisting of removing cells of Pichia fermentans, the proteins, the lipids and the mannans by alkali treatment, then the glycogen and the soluble glucans by acid treatment.

5. Material based on extracts according to claims 2 or 4, characterised by the fact that the extracts are principally composed of insoluble glucans.

6. Material according to claim 5, characterised in that the insoluble glucans are essentially of β-(1-3)-D-glucans.

7. Pharmaceutical composition comprising as active material Pichia or extracts of Pichia such as defined in any one of claims 1 to 6, as well as a pharmaceutically acceptable vehicle.

8. Pharmaceutical composition according to claim 7, which is orally administrable, characterised by the fact that it contains whole cells of Pichia or of extracts of Pichia.

9. Pharmaceutical composition according to claim 7, which is parenterally administrable, characterised by the fact that it contains insoluble glucans of Pichia.

10. Pharmaceutical composition according to claim 7, in aerosol form, characterised by the fact that it contains insoluble glucans of Pichia.

## Ansprüche

1. Substanz, die geeignet ist zur Behandlung von Infektionskrankheiten, zur nichtspezifischen Immunostimulation, zur Verstärkung der Wirkung von Antibiotika und zur Erhöhung der Wirkung von Vaccinen, dadurch gekennzeichnet, daß sie aus Pichia oder aus Pichia-Extrakten besteht.

2. Substanz nach Anspruch 1, dadurch gekennzeichnet, daß sie aus Pichia fermentans oder aus Pichia fermentans-Extrakten besteht.

3. Substanz nach Anspruch 2, dadurch gekennzeichnet, daß sie aus ganzen Zellen von Pichia fermentans besteht.

4. Substanz nach Anspruch 2, dadurch gekennzeichnet, daß sie aus Zellwand-Extrakten von Pichia fermentans besteht, die nach einem Verfahren erhalten worden sind, welches darin besteht, daß man die Zellmassen von Pichia fermentans, die Proteine und die Lipide und die Mannane durch alkalische Behandlung and danach das Glykogen und die löslichen Glucane durch saure Behandlung eliminiert.

5. Substanz in Form eines Extrakts gemäß einem der Ansprüche 2 oder 4, dadurch gekennzeichnet, daß der Extrakt im wesentlichen aus unlöslichen Glucanen besteht.

6. Substanz nach Anspruch 5, dadurch gekennzeichnet, daß diese unlöslichen Glucane im wesentlichen β-(1-3)-D-Glucane sind.

7. Pharmazeutisches Präparat, dadurch gekennzeichnet, daß es als wirksame Substanz Pichia oder Pichia-Extrakte gemäß einem der Ansprüche 1 bis 6, sowie einen pharmazeutisch akzeptablen Träger enthält.

8. Pharmazeutisches Präparat nach Anspruch 7, welches auf oralem Wege verabreichbar ist, dadurch gekennzeichnet, daß es ganze Zellen von Pichia oder Pichiaextrakte enthält.

9. Pharmazeutisches Präparat nach Anspruch 7, das auf parenteralem Wege verabreichbar ist, dadurch gekennzeichnet, daß es unlösliche Glucane aus Pichia enthält.

10. Pharmazeutisches Präparat nach Anspruch 7 in Form eines Aerosols, dadurch gekennzeichnet, daß es unlösliche Glucane aus Pichia enthält.